# EUROPEAN PATENT SPECIFICATION

(11) **EP 1 243 260 B1**
(45) Date of publication and mention of the grant of the patent: **15.06.2005**
(21) Application number: 02006344.2
(22) Date of filing: 21.03.2002
(51) Int. Cl.: A61K 9/70, A61L 15/28, A61L 15/58, A61L 15/00

(54) **Cicatrizant hydrocolloidal patch containing hyaluronic acid and chondroitin sulphate**
Narbenbildung fördernder hydrokolloidaler Wundverband, welcher Hyaluronsäure und Chondroitinsulfat enthält
Pansement hydrocolloidal cicatrisant contenant de l'acide hyaluronique et du sulfate de chondroitine

(30) Priority: 22.03.2001 IT MI010611
(43) Date of publication of application: 25.09.2002
(73) Proprietor: Ibsa Institut Biochimique S.A., 6900 Lugano (CH)
(72) Inventor: Garavani, Alberto, 6900 Massagno (CH); Rapaport, Irina, 6849 Rovio (CH)
(74) Representative: Gervasi, Gemma, Dr.

(56) References cited:
- EP-A- 0 302 536
- DE-A- 19 712 699
- DATABASE MEDLINE [Online] September 1989 (1989-09) FIALKOVA M A ET AL: "[The effect of chondroitin sulfate preparations on wound healing and the strength of the surgical scar]" Database accession no. NLM2514813 XP002202622 & BIULLETEN' EKSPERIMENTAL'NOI BIOLOGII I MEDITSINY. USSR SEP 1989, vol. 108, no. 9, September 1989 (1989-09), pages 350-351, ISSN: 0365-9615
- SUYAMA T ET AL: "The acceleration of wound healing with chondroitin sulfate A and its acidic hydrolysates." THE JAPANESE JOURNAL OF EXPERIMENTAL MEDICINE. JAPAN AUG 1966, vol. 36, no. 4, August 1966 (1966-08), pages 449-452, XP002202620 ISSN: 0021-5031
- CHEN W Y ET AL: "Functions of hyaluronan in wound repair." WOUND REPAIR AND REGENERATION: OFFICIAL PUBLICATION OF THE WOUND HEALING SOCIETY [AND] THE EUROPEAN TISSUE REPAIR SOCIETY. UNITED STATES 1999 MAR-APR, vol. 7, no. 2, March 1999 (1999-03), pages 79-89, XP002202621 ISSN: 1067-1927

## Description

### FIELD OF THE INVENTION

The present invention concerns a cicatrizant hydrocolloidal patch and the relative preparation process.

### STATE OF THE ART

Wound dressings of various types, eg. as described in EP-A-302 536 are known in the art.

Cicatrizant pharmaceutical formulations for topical use, based on hyaluronic acid or a pharmaceutical salt thereof, have also been known for some time.

For instance EP 0480198 describes pharmaceutical compositions containing the sodium salt of hyaluronic acid and antiseptic substances for topical use.

However, these compositions are in hydrogel form and have the disadvantage of adding liquid to the wound when applied to it, hence making it even more difficult to eliminate the exudate from the wound.

These drawbacks are solved with the self-supporting dry transparent film described in international patent application WO 97/02845, consisting of a mixture of at least one hydrocolloid and hyaluronic acid.

This film is prepared with a process that envisages the following steps:
· preparation of a very diluted aqueous composition containing hyaluronic acid at concentrations between 0.5 and 2% in weight, and hydrocolloids at concentrations between 1% and 20% optionally in the presence of an additional solvent such as glycerol.
· casting of the composition on a support,
· exsiccation of all the liquid components of the aforesaid composition by treatment in a stove.

An anhydrous film is thus obtained in which the content of hyaluronic acid is between 2 and 98% and the remaining components to 100% are the hydrocolloids. The complete elimination of the water, achieved by exsiccation in a stove requires extremely long heating times (in the order of several hours), with a consequent notable waste of energy. In addition, since with this type of procedure it is difficult to obtain a film of uniform thickness with a constant level of anhydricity, the industrial implementation of such a process is virtually impracticable.

Moreover, all the hydrocolloids, with the exclusion of polyvinylpyrrolidone, tend to worsen the mechanical properties of the self-supporting film, namely its tensile strength and elongation at break. To obviate such a drawback large quantities of the expensive hyaluronic acid must be added, in most cases, more than 10% and, in some cases, in the event that sodium alginate is used as the hydrocolloid, in amounts decidedly greater than 25%. Only when polyvinylpyrrolidone is employed it is possible to obtain good mechanical properties using smaller amounts of hyaluronic acid, which in any case must be around 2.5%.

DE 197 12 699 is another example for a plaster whose active film comprises at least 2% or more of hyaluronic acid.

In Italian patent 1301470 a cicatrizant hydrocolloidal patch is described comprising a support layer, an intermediate layer containing an adhesive polymer, at least one hydrocolloid and hyaluronic acid or a pharmaceutical salt thereof and, finally, a protective layer.

This patch does not show sufficient cicatrizant strength even at concentrations of hyaluronic acid in the order of 2% in weight out of the weight of the adhesive layer. In fact, the cicatrizant effect does not diverge, in a statistically significant manner, from the cicatrizant activity shown by the placebo patch, not containing any active principle.

In "Effect of chondroitin sulfate preparation on wound healing and strength of the surgical scar" by M. Fialkova et al BYULLETTIN EKSPERIMENTAL' NOY BIOLOGII I MEDITSINY, Vol. 108, N°9 pp. 350-351 the results of an experiment carried out on a model of "full thickness" (300 mm²) cutaneous wound in rats are discussed, wherein such a wound has been treated with one or two applications of 30 mg of sodium chondroitin sulphate. The reduction of the damaged surface (measuring through planimetry) was more rapid in the treated group. For instance, 8 days after the second application the residual area in the treated group was half that of the control group, and also the clinical signs connected with the lesion (edema-exudate) decreased more quickly in the treated group.

The therapeutic form considered in this article diverges from that considered in the Italian patent since the active principle is administered intramuscularly by means of an injectable solution and at high concentrations of 10%.

### SUMMARY OF THE INVENTION

The Applicant has now unexpectedly discovered a cicatrizant hydrocolloidal patch containing the sodium salt of hyaluronic acid and sodium chondroitin sulphate as the active principle which, at low concentrations of both sodium chondroitin sulphate and sodium salt of hyaluronic acid, when it is applied to a wound allows to attain a cicatrization speed, expressed as percentage of reduction of the wound surface in time, comparable to that of bandages available on the market for the same purposes, namely CONVATEC® or VARIHESIVE-E®, but, unlike the latter, also promotes the formation of dermis and collagen production.

The object of the present invention is therefore a cicatrizant hydrocolloidal patch comprising:
a) a support layer,
b) an adhesive layer containing an adhesive polymer, at least one hydrocolloid, the sodium salt of hyaluronic acid in a concentration of between 0,05% and 1% in weight of the said adhesive layer and sodium chondroitin sulphate in a concentration of between 0,05% and 1% in weight of the said adhesive layer.
c) a protective layer removable at the moment of use.

### DETAILED DESCRIPTION OF THE INVENTION

The patch object of the present invention contains the sodium salt of hyaluronic acid whose molecular weight, expressed as hyaluronic acid, is preferably between 50,000 and 1,000,000.

The sodium chondroitin sulphate in the patch of the present invention is preferably the bisodium salt of chondroitin-4-sulphate or chondroitin sulphate A.

According to the present invention, the concentration of sodium hyaluronate in the patch is between 0.05 and 1%, and that of sodium chondroitin sulphate is between 0.05 and 1%.

In fact, it has surprisingly been found that, when such active principles have concentrations that fall within the aforesaid preferred intervals and particularly when sodium hyaluronate has a concentration of 0.2% and sodium chondroitin sulphate has a concentration of 0.3% in weight out of the total weight of adhesive layer (b), the patch of the invention shows a greater cicatrizant effect compared to that a patch of similar formulation, but containing sodium hyaluronate at a concentration of 2% and sodium chondroitin sulphate at a concentration of 3% in weight out of the total weight of the adhesive layer, and comparable to that of bandages available on the market such as VARIHESIVE®.

Preferred hydrocolloids for use in adhesive layer (b) of the patch according to the present invention are sodium carboxymethylcellulose of molecular weight of between 700 and 50,000, pectin USPL optionally mixed with saccharose, or mixtures thereof.

The concentration of said hydrocolloid is preferably between 10 and 90% in weight out of the total weight of adhesive layer (b).

According to a particularly preferred solution, a mixture of the following is used as a hydrocolloid: sodium carboxymethylcellulose, commercially available under the trade name of Blancosa® 7H4XF, sodium carboxymethylcellulose commercially available under the trade name of CEKOL®, Pectin USPL available under the trade name of GENU-PECTIN, added with saccharose (Sugar mix). This mixture of hydrocolloids is preferably present in adhesive layer (b) at concentrations of between 10 and 80%, even more preferably at concentrations of 47% in weight out of the total weight of said adhesive layer (b).

The adhesive polymer of layer (b) of the patch object of the present invention is preferably chosen between polyisobutylene of molecular weight of between 500 and 100,000, isoprene/styrene copolymer or mixtures thereof, at concentrations of between 10 and 90% in weight out of the total weight of adhesive layer (b).

According to a particularly preferred solution a mixture of polyisobutylene having a mean molecular weight of 40,000 and commercially available under the trade name of Oppanol® B15, and of styrene/isoprene copolymer Kraton® D-1107CS is used. The concentration of said adhesive polymeric mixture in layer (b) is preferably between 10 and 80%, even more preferably of 45% in weight out of the total weight of the adhesive layer (b).

The patch according to the present invention preferably contains a plasticizer chosen in the group consisting of mineral oil optionally with traces of white naphthenic oil, commercially available under the trade name of ENERPAR® and a mixture of polyterpenic resin and petroleum hydrocarbon resin, commercially available under the trade name of WINGTAC®10, and relative mixtures of said plasticizers at concentrations of between 0.5 and 25% in weight calculated out of the total weight of said adhesive layer (b). According to a preferred solution a mixture of the aforesaid mineral oil and of the mixture of polyterpenic resin/petroleum hydrocarbon resin is used and the total concentration of said plasticizer is between 1 and 10%, and even more preferably of 8% in weight out of the total weight of the adhesive layer (b).

In the patch according to the present invention preferably the support or layer (a) is made up of polyurethane as a film or a foam, while layer (c), which is the sheet removable at the moment of use, is preferably made of silicon paper.

The patch object of the present invention is preferably produced with a process that comprises the following steps:
i) dry mixing of the sodium salf of hyaluronic acid and the sodium chondroitin sulphate with the hydrocolloid,
ii) mixing of the powders of the previous stage with the adhesive composition and optionally a plasticizer;
iii) extrusion of the paste deriving from step (ii) at a temperature of between 40 and 90°C, preferably of 80°C, between the support layer (a) and the removable protective layer (c).

Shown below are two illustrative but non-limiting examples of composition of the hydrocolloidal patch according to the present invention.

### EXAMPLE 1

### Hydrocolloidal patch composed of:

1. layer (c): silicon paper = 0.82 g/total weight of the patch
2. layer (b): adhesive = 10.25g/total weight of the patch,
3. layer (a): polyurethane support film = 0.62 g/total weight of the patch.

### Composition of adhesive layer (b)

| Trade name | Usual name | % in weight out of the total weight of layer (b) |
|---|---|---|
| OPPANOL® B15 | Polyisobutylene | 29.24 |
| KRATON®D-1107CS | Styrene-isoprene copolymer | 15.59 |
| BLANCOSA® 7H4XF | Sodium carboxymethylcellulose | 17.55 |
| GENU-PECTIN | Pectin USPL | 11.70 |
| CEKOL®4000 | Sodium carboxymethylcellulose | 15.59 |
| SUGARMIX® | Saccharose | 1.95 |
| WINGTAC®10 | Synthetic polyterpenic resin/ petroleum hydrocarbon resin | 3.90 |
| ENERPAR® | Mineral oil with traces of white naphthenic oil | 3.90 |
| Sodium hyaluronate | | 0.23 |
| Sodium chondroitin sulphate | | 0.35 |

### EXAMPLE 2

| Trade name | Usual name | % in weight out of the total weight of layer (b) |
|---|---|---|
| OPPANOL® B15 | Polyisobutylene | 27.78 |
| KRATON®D-1107CS | Styrene-isoprene copolymer | 14.82 |
| BLANCOSA ® 7H4XF | Sodium carboxymethylcellulose | 16.67 |
| GENU-PECTIN | Pectin USPL | 11.11 |
| CEKOL®4000 | Sodium carboxymethylcellulose | 14.82 |
| SUGARMIX® | Saccharose | 1.85 |
| WINGTAC®10 | Synthetic polyterpenic resin/ petroleum hydrocarbon resin | 3.70 |
| ENERPAR® | Mineral oil with traces of white naphthenic oil | 3.70 |
| Sodium hyaluronate | | 2.22 |
| Sodium chondroitin sulphate | | 3.33 |

After cutting, each patch was sealed in a special airtight blister pack and irradiated with γ rays (normally between 25 and 50 KGy)
1- MACROSCOPIC APPEARANCE OF THE WOUNDS AND MORPHOMETRIC ANALYSIS

### METHODOLOGY

Dunkin Hartley type guinea pigs were used for this test.
A rectangular wound of 12 cm² (4x3) was made on one side of each guinea pig (10 guinea pigs + 1 extra per group), maintaining the panniculus carnosus.

The medication was applied to the wound each day up to day 33 (end of the experiment).

The action of the medication and the appearance of the wounds were macroscopically analyzed following a scale of criteria based on moisture, adherence to the wound, inflammatory and haemorrhagic process and level of cicatrization.

A photograph was taken every two or three removals of the medication under standard conditions in order to automatically highlight the progress of the surface of the wound with an image analyser.

The following types of medication were tested.
- group A: placebo hydrocolloidal patch
- group B: hydrocolloidal plasters of example 1
- group C: hydrocolloidal plaster of example 2
- group D: VARIHESIVE ® patch

### Results

### a) MACROSCOPIC APPEARANCE OF THE WOUNDS

The wounds of groups A, B, and C were moist, dark and sanguinolent for most of the time, whereas the wounds of group D were moist but not as dark as those in the other groups and also less sanguinolent. However, in the latter case some yellow liquid was observed in the wounds after day 5.

A certain tendency towards better cicatrization was observed in group B when compared to group A. In fact, the wounds in group B were smaller and had a better macroscopic aspect, since covered by a thinner scab.

### b- MORPHOMETRIC ANALYSES OF THE SURFACE OF THE WOUNDS

### b-1 Surface of the skin of the different groups

**Table I**

| evolution of the mean surface of the wound in different groups (cm²) | | | | |
|---|---|---|---|---|
| Day | GROUP A | GROUP B | GROUP C | GROUP D |
| 1 | 13.60 | 13.89 | 13.41 | 12.83 |
| 5 | 10.26 | 9.58 | 9.59 | 8.87 |
| 7 | 9.88 | 8.27 | 9.34 | 6.76 |
| 11 | 6.73 | 5.45 | 5.41 | 4.92 |
| 15 | 5.01 | 3.99 | 4.57 | 3.74 |
| 19 | 4.27 | 3.21 | 3.82 | 3.10 |
| 24 | 3.17 | 2.54 | 3.15 | 2.37 |
| 28 | 2.87 | 2.30 | 2.82 | 2.32 |
| 31 | 2.54 | 1.83 | 2.49 | 2.33 |
| 33 | 2.48 | 1.72 | 2.38 | 1.72 |

The results reported above show that in all groups the surface of the wound was reduced of approximately 50% in the first 11 days and was reduced more slowly afterwards.

A difference in the speed of cicatrization is observed between the different groups:
- 50% cicatrization of the surface for groups B and D at 8.3 and 8 days respectively, while in groups A and C this cicatrization is obtained at 10.9 and 9.4 days respectively.
- 75% cicatrization of the surface of the skin for groups B and D at 18.6 and 18.9 days respectively, while for groups A and C this value is reached at 23.8 and 23.7days respectively.

The speed in cicatrization is better in groups B and D.

### b-2- Statistical analysis

Statistical analysis of the residual surface (non-parametric Mann & Whitney test) did not show any significant difference (p< 0.05) between groups B and D, while cicatrization in groups A and C showed the same course.

### 2- HISTOLOGICAL EXAMINATION

### METHODOLOGY

The experiment was stopped at day 33 and the skin of three animals was taken for each group. After fixing with 10% formaldehyde, the samples were then denatured in alcohol solutions of increasing concentration and afterwards incorporated in paraffin.

Two pairs in a series, of approx. 5 µm thickness, were made on each sample with the aid of a HM350 microtome. The sections were dyed according to a modified trichromium technique for classic histopathological analysis and with toluidine blue to highlight metachromatic oxydic structures.

The pairs of histological samples were observed using a Polyvar microscope (Reichert) fitted with a 4, 10 and 25 objective with the possibility of adding a 1.25 lens.

### RESULTS

The wounds of the animals of group A and group D give similar results from a histological point of view.

Hypervascularization and exudates consisting of red blood cells and the presence of a foamy collagen-based matrix in the deep layers of the granulation tissue, were found in both groups. However, group A was associated with a more marked hypervascularization and inflammatory component when compared to group D.

The wounds of groups B and C show similar characteristics with respect to those of groups A and D, but, in addition, in the former ones the presence of a more mature deep dermis is observed. The collagen is denser and very similar to the adjacent layer of the normal dermis. This is particularly marked for the wounds of group C.

However, this group also shows a relative superficial granulation tissue of inflammatory type with a weakening of the epidermization process when compared to the wounds of group B.

Therefore, the active principles of the patches applied to groups B and C show a twoford effect, that is:
1) improved synthesis of collagen and organization in the deep layers of the wounds of groups (B) and in particular (C),
2) a more marked superficial inflammatory component associated with these active principles in the case of the groups (C).

## Claims

1. Cicatrizant hydrocolloidal patch comprising
(a) a support layer,
(c) an adhesive layer containing an adhesive polymer, at least one hydrocolloid, the sodium salt of hyaluronic acid in a concentration of between 0,05% and 1% in weight of the said adhesive layer and sodium chondroitin sulphate in a concentration of between 0,05% and 1% in weight of the said adhesive layer,
(c) a protective layer removable at the moment of use.

2. The patch as in claim 1, **characterized in that** the molecular weight of the hyaluronic acid sodium salt, expressed as hyaluronic acid, is between 50,000 and 1,000,000.

3. The plaster as in claim 2, **characterized in that** the concentration of sodium hyaluronate is 0.2% and that of sodium chondroitin sulphate is 0.3% out of the total weight of adhesive layer (b).

4. The patch as in any one of claims 1-3, **characterized in that** the hydrocolloid is chosen from the group consisting of sodium carboxymethylcellulose of molecular weight of between 700 and 50,000, pectin USPL optionally mixed with saccharose, or relative mixtures thereof.

5. The patch as in claim 4, **characterized in that** the concentration of said hydrocolloid is between 10 and 90% in weight out of the total weight of adhesive layer (b).

6. The patch as in any one of claims 4 and 5, **characterized in that** said hydrocolloid is a mixture of sodium carboxymethylcellulose and pectin USPL added with saccharose at concentrations of between 10 and 80% in weight out of the total weight of adhesive layer (b).

7. The patch as in claim 6, in which the concentration of said hydrocolloid is equal to 47% in weight.

8. The patch as in any one of claims 1-7, **characterized in that** the adhesive polymer of layer (b) is chosen between polyisobutylene of molecular weight of between 500 and 100,000, isoprene/styrene copolymer, or relative mixtures thereof, at concentrations of between 10 and 90% in weight out of the total weight of adhesive layer (b).

9. The patch as in claim 8, **characterized in that** the adhesive polymer of layer (b) consists of a mixture of polyisobutylene with a molecular weight of 40,000 and of styrene/isoprene copolymer at a concentration of between 10 and 80% in weight out of the total weight of adhesive layer (b).

10. The patch as in claim 9, **characterized in that** the concentration of said polymeric mixture is equal to 45% in weight out of the total weight of adhesive layer (b).

11. The patch as in any one of claims 1-10, **characterized in that** it contains a plasticizer chosen from the group consisting of mineral oil optionally with traces of white naphthenic oil, a mixture of petroleum hydrocarbon resin and polyterpenic resin, and relative mixtures of said plasticizers at concentrations of between 0.5 and 25% in weight calculated out of the total weight of said adhesive layer (b).

12. The patch as in claim 11, **characterized in that** said plasticizer is a mixture of said mineral oil with said mixture of polyterpenic resin/petroleum hydrocarbon resin and the total concentration of said plasticizer is between 1 and 10% in weight out of the total weight of adhesive layer (b).

13. The patch as in claim 12, **characterized in that** the concentration of said plasticizer is 8% in weight out of the total weight of adhesive layer (b).

14. The patch as in any one of claims 1-13, **characterized in that** the support layer (a) consists of polyurethane as a film or foam.

15. The patch as in any one of claims 1-14, **characterized in that** layer (c), the sheet removable at the moment of use, is preferably made of silicon paper.

16. Process to prepare the patch as in any one of claims 1-15 comprising the following steps:
i) dry mixing of the sodium salt of hyaluronic acid and the sodium chondroitin sulphate with the hydrocolloid,
ii) mixing of the powders of the previous stage with the adhesive composition and optionally a plasticizer;
iii) extrusion of the paste deriving from step (ii) at a temperature of between 40 and 90°C between support layer (a) and the removable protective layer (c).

17. The process as in claim 16, **characterized in that** the temperature of stage (iii) is 80°C.

## Patentansprüche

1. Hydrokolloidaler Cicatrizant enthaltender Wundverband umfassend:
(a) eine Trägerschicht,
(c) eine adhäsive Schicht bestehend aus einem adhäsiven Polymer, mindestens einem Hydrokolloid, dem Natriumsalz der Hyaluronsäure in einer Konzentration von 0,05% bis 1 Gew.% bezogen auf die genannte adhäsive Schicht und Natriumchondroitinsulfat in einer Konzentration von 0,05 bis 1 Gew% der genannten adhäsiven Schicht,
(c) eine protektive Schicht, die vor dem Gebrauch entfernt wird.

2. Wundverband gemäß Anspruch 1, **dadurch gekennzeichnet, dass** das Molekulargewicht des Natriumsalzes der Hyaluronsäure, ausgedrückt als Hyaluronsäure zwischen 50.000 und 1.000.000 beträgt.

3. Wundverband gemäß Anspruch 2, **dadurch gekennzeichnet, dass** die Konzentration an Natriumhyaluronat 0,2 Gew% und die Konzentration von Natriumchondroitinsulfat 0,3 Gew% bezogen auf das Gesamtgewicht der adhäsiven Schicht ausmacht.

4. Wundverband gemäß einem der Ansprüche von 1-3, **dadurch gekennzeichnet, dass** das Hydrokolloid ausgewählt wird aus der Gruppe bestehend aus Natriumcarboxymethylcellulose mit einem Molekulargewicht zwischen 700 und 50.000 und Pektin USPL optional vermischt mit Saccharose oder entsprechenden Mischungen daraus.

5. Wundverband gemäß Anspruch 4, **dadurch gekennzeichnet, dass** die Konzentration an genanntem Hydrokolloid 10 bis 90 Gew% bezogen auf das Gesamtgewicht der adhäsiven Schicht (b) beträgt.

6. Wundverband gemäß einem der Ansprüche 4 oder 5, **dadurch gekennzeichnet, dass** das Hydrokolloid eine Mischung aus Natriumcarboxymethylcellulose und Pektin USPL ist, der Saccharose zugegeben wird, und welches in einer Konzentration von 10 bis 80 Gew% bezogen auf das Gesamtgewicht der adhäsiven Schicht (b) vorliegt.

7. Wundverband gemäß Anspruch 6, bei welchem die Konzentration an genanntem Hydrokolloid gleich 47 Gew% entspricht.

8. Wundverband gemäß einem der Ansprüche 1-7, **dadurch gekennzeichnet, dass** das adhäsive Polymer der Schicht (b) ausgewählt wird unter Polyisobutylen mit einem Molekulargewicht zwischen 500 und 100.000, Isopren/Styrol-Copolymer oder entsprechenden Mischungen daraus in Konzentrationen von 10 bis 90 Gew% bezogen auf das Gesamtgewicht der adhäsiven Schicht.

9. Wundverband gemäß Anspruch 8, **dadurch gekennzeichnet, dass** das adhäsive Polymer der Schicht (b) aus einer Mischung von Polyisobutylen mit einem Molekulargewicht von 40.000 und Styrol/Isopren-Copolymer mit einer Konzentration von 10 bis 80 Gew% bezogen auf das Gesamtgewicht der adhäsiven Schicht (b) besteht.

10. Wundverband gemäß Anspruch 9, **dadurch gekennzeichnet, dass** die Konzentration an genannter Polymermischung 45 Gew% bezogen auf das Gesamtgewicht der adhäsiven Schicht (b) beträgt.

11. Wundverband gemäß einem der Ansprüche 1-10, **dadurch gekennzeichnet, dass** er einen Weichmacher enthält ausgewählt aus der Gruppe bestehend aus Mineralöl optional mit Spuren an Naphthen-Öl, einer Mischung aus Petroleumhydrocarbonharz und Polyterpenharz sowie entsprechenden Mischungen aus den genannten Weichmachern in Konzentrationen von 0,5 bis 25 Gew% bezogen auf das Gesamtgewicht der genannten adhäsiven Schicht (b).

12. Wundverband gemäß Anspruch 11, **dadurch gekennzeichnet, dass** genannter Weichmacher aus einer Mischung von genanntem Mineralöl mit genannter Mischung aus Polyterpenharz/Petroleumhydrocarbonharz besteht und die Gesamtkonzentration des genannten Weichmachers einen Anteil von 1 bis 10 Gew% bezogen auf das Gesamtgewicht der adhäsiven Schicht hat.

13. Wundverband gemäß Anspruch 12, **dadurch gekennzeichnet, dass** die Konzentration an genanntem Weichmacher 8 Gew% bezogen auf das Gesamtgewicht der adhäsiven Schicht (b) beträgt.

14. Wundverband gemäß einem der Ansprüche 1-13, **dadurch gekennzeichnet, dass** die Trägerschicht (a) aus Polyurethan in Form eines Films oder Schaums besteht.

15. Wundverband gemäß einem der Ansprüche 1-14, **dadurch gekennzeichnet, dass** die Schicht (c), die vor Gebrauch entfernt wird, vorzugsweise aus Silikonpapier besteht.

16. Verfahren zur Herstellung von Wundverband gemäß einem der Ansprüche 1-15, welches die folgenden Schritte umfasst:
i) Trockenmischung aus Natriumsalz der Hyaluronsäure und Natriumchondroitinsulfat mit dem Hydrokolloid,
ii) Mischung der Pulver aus vorherigem Schritt mit dem adhäsiven Bestandteil und optional mit einem Weichmacher,
iii) Extrusion der aus Schritt (ii) erhaltenen Paste bei einer Temperatur von 40 bis 90°C zwischen Trägerschicht (a) und der entfernbaren protektiven Schicht (c).

17. Verfahren gemäß Anspruch 16, **dadurch gekennzeichnet, dass** die Temperatur in Schritt (iii) 80°C beträgt.

## Revendications

1. Patch hydrocolloïdal cicatrisant comprenant
(a) une couche de support,
(c) une couche adhésive contenant un polymère adhésif, au moins un hydrocolloïde, le sel de sodium de l'acide hyaluronique à une concentration comprise entre 0,05% et 1% en poids de ladite couche adhésive et du sulfate de chondroïtine sodium à une concentration comprise entre 0,05% et 1% en poids de ladite couche adhésive,
(c) une couche protectrice pouvant être enlevée au moment de l'utilisation.

2. Patch selon la revendication 1, **caractérisé en ce que** le poids moléculaire du sel de sodium de l'acide hyaluroniqùe, exprimé en tant qu'acide hyaluronique, est compris entre 50.000 et 1.000.000.

3. Emplâtre selon la revendication 2, **caractérisé en ce que** la concentration en hyaluronate de sodium est de 0,2% et **en ce que** le sulfate de chondroïtine sodium représente 0,3% du poids total de la couche adhésive (b).

4. Patch selon l'une quelconque des revendications 1-3, **caractérisé en ce que** l'hydrocolloïde est choisi dans le groupe consistant en sodium carboxyméthylcellulose d'un poids moléculaire compris entre 700 et 50.000, pectine USPL facultativement en mélange avec du saccharose ou leurs mélanges relatifs.

5. Patch selon la revendication 4, **caractérisé en ce que** la concentration dudit hydrocolloïde est comprise entre 10 et 90% en poids sur le poids total de la couche adhésive (b).

6. Patch selon l'une quelconque des revendications 4 et 5, **caractérisé en ce que** ledit hydrocolloïde est un mélange sodium carboxyméthylcellulose et de pectine USPL additionné de saccharose à des concentrations comprises entre 10% et 80% en poids du poids total de la couche adhésive (b).

7. Patch selon la revendication 6, dans lequel la concentration dudit hydrocolloïde est égale à 47% en poids.

8. Patch selon l'une quelconque des revendications 1-7, **caractérisé en ce que** le polymère adhésif de la couche (b) est choisi entre polyisobutylène d'un poids moléculaire compris entre 500 et 100.000, copolymère d'isoprène / styrène ou leurs mélanges relatifs, à des concentrations comprises entre 10 et 90% en poids sur le poids total de la couche adhésive (b).

9. Patch selon la revendication 8, **caractérisé en ce que** le polymère adhésif de la couche (b) consiste en un mélange de polyisobutylène avec un poids moléculaire de 40.000 et d'un copolymère de styrène / isoprène à une concentration comprise entre 10 et 80% en poids sur le poids total de la couche adhésive (b).

10. Patch selon la revendication 9, **caractérisé en ce que** la concentration dudit mélange polymérique est égale à 45% en poids sur le poids total de la couche adhésive (b).

11. Patch selon l'une quelconque des revendications 1-10, **caractérisé en ce qu'**il contient un plastifiant choisi dans le groupe consistant en huile minérale éventuellement avec des traces d'huile naphténique blanche, un mélange de résine d'hydrocarbure de pétrole et de résine polyterpénique et des mélanges relatifs desdits plastifiants à des concentrations comprises entre 0,5 et 25% en poids en calculant sur le poids total de ladite couche adhésive (b).

12. Patch selon la revendication 11, **caractérisé en ce que** ledit plastifiant est un mélange de ladite huile minérale avec ledit mélange de résine polyterpénique / résine d'hydrocarbure de pétrole et la concentration totale dudit plastifiant est comprise entre 1 et 10% en poids sur le poids total de la couche adhésive (b).

13. Patch selon la revendication 12, **caractérisé en ce que** la concentration dudit plastifiant est de 8% en poids sur le poids total de la couche adhésive (b).

14. Patch selon l'une quelconque des revendications 1-13, **caractérisé en ce que** la couche de support (a) consiste en polyuréthanne, sous la forme d'un film ou d'une mousse.

15. Patch selon l'une quelconque des revendications 1-14, **caractérisé en ce que** la couche (c), la feuille pouvant être ôtée au moment de l'utilisation, est de préférence faite d'un papier de silicone.

16. Procédé de préparation du patch de l'une quelconque des revendications 1-15 comprenant les étapes suivantes:
i) mélange à sec du sel de sodium de l'acide hyaluronique et du sulfate de chondroïtine sodium avec l'hydrocolloïde,
ii) Mélange des poudres du stade précédent avec la composition adhésive et facultativement un plastifiant,
iii) Extrusion de la pâte dérivant de l'étape (ii) à une température comprise entre 40 et 90°C entre la couche de support (a) et la couche protectrice (c) pouvant être ôtée.

17. Procédé selon la revendication 16, **caractérisé en ce que** la température du stade (iii) est de 80°C.
